(19) Europäisches Patentamt
European Patent Office
Office européen des brevets

(11) Publication number: 0 053 018 B1

(12) EUROPEAN PATENT SPECIFICATION

(45) Date of publication of patent specification: 05.09.84

(21) Application number: 81305493.9

(22) Date of filing: 20.11.81

(51) Int. Cl.³: C 01 B 21/06, C 01 B 31/30, B 01 J 23/28, B 01 J 27/00, C 01 C 1/04, C 07 C 1/04, C 07 C 1/10

# (54) Novel molybdenum oxycarbonitride compositions.

(30) Priority: 24.11.80 US 209998
15.12.80 US 216139
15.12.80 US 216542
16.03.81 US 243824
23.09.81 US 304980

(43) Date of publication of application: 02.06.82 Bulletin 82/22

(45) Publication of the grant of the patent: 05.09.84 Bulletin 84/36

(84) Designated Contracting States: BE DE FR GB NL

(56) References cited:
FR-A-2 418 209
US-A-3 416 891
US-A-3 492 100
US-A-3 872 136

(73) Proprietor: Exxon Research and Engineering Company
P.O.Box 390 180 Park Avenue
Florham Park New Jersey 07932 (US)

(72) Inventor: McCandlish, Larry Eugene
25 Harrison Avenue
Highland Park New Jersey (US)
Inventor: Turaew, Larissa Wasilivna
6-17 Third Street
Fairlawn New Jersey (US)
Inventor: Wright, Franklin John
75 Oakwood Road
Watchung New Jersey (US)
Inventor: Kugler, Edwin Lee
58 Tall Oaks Drive
Summit New Jersey (US)

(74) Representative: Field, Roger Norton et al
ESSO Engineering (Europe) Ltd. Patents & Licences Apex Tower High Street
New Malden Surrey KT3 4DJ (GB)

EP 0 053 018 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a new composition of matter, face-centered, cubic molybdenum oxycarbonitride, a general method for its preparation, and its utility as a catalyst in producing hydrocarbons from the reaction of CO and $H_2$, and CO and steam, and also in catalytically producing ammonia from the reaction of $N_2$ and $H_2$.

CO hydrogenation, including the Fischer-Tropsch synthesis is well-known in the art for producing a broad range of gaseous liquid and solid hydrocarbons including methane ($C_1$) to $C_{20}$ and higher carbon chain numbers. The process usually uses iron-based catalysts promoted with various agents such as potassium carbonate and the like to improve product distribution and selectivity.

A related process, CO hydrogenation with steam, is well-known in the art and is generally referred to as the Kolbel-Engelhardt process. In the process, mixtures of CO and steam are usually contacted with an iron-based catalyst at high temperature and pressure resulting in a broad hydrocarbon distribution from $C_1$ to $C_{20}$ carbon number, including gaseous, liquid and solid products, and also including high molecular weight waxes.

Furthermore, the synthesis of ammonia from contacting mixtures of hydrogen and nitrogen with a suitable catalyst is well-known in the art. Examples of such processes are the Haber process, modified Haber-Bosch process, Claude process, Casale process, Fauser process and Mont Cenis process. At present, all of these processes generally utilize promoted iron catalysts.

There is a constant search in the field for new types of catalysts and catalyst combinations that are potentially less expensive, give better conversions, have greater longevity and better catalyst selectivity and activity in the above-described processes.

One field of compositions in which new catalysts are currently being searched for are the metal carbides. Already known in this art are carbides such as tungsten and molybdenum carbides, various oxycarbides and carbonitrides of Group VIA metals. In addition, several general references to oxycarbonitrides of Group VIA metals are also known in the art.

*U.S. Patent 3,492,100 (1970)* describes the general preparation of oxycarbonitrides and carbonnitrides of metals from Group IVA, VA, and mixed metal oxycarbonitrides and carbonitrides of Group VIA metals. Particularly described are those of titanium, zirconium, vanadium, niobium, and tantalum.

*Japanese Kokai 10,692 (1978)* describes coated hard alloys for cutting tools comprising an intermediate layer of a Group IVA, VA or VIA oxycarbonitride.

*USP 3,872,136 (1975)* discloses a process for the preparation of vanadium carbonitride, vanadium oxycarbide or vanadium oxycarbonitride from other vanadium compounds.

The reference *Izv. Akad. Nauk. SSSR, Neorg Mater. 1976, 12(9), 1581—4,* describes cubic oxycarbonitrides of Ti and Zn and methods of preparation.

However, the above-described references do not contain working examples of bulk molybdenum oxycarbonitride, or to a specific method for its preparation in bulk or catalyst form, or to its use as a catalyst in hydrocarbon synthesis from CO and $H_2$, or CO and steam, or catalyzed $NH_3$ synthesis from $N_2$ and $H_2$.

It has unexpectedly been found that face-centered, cubic molybdenum oxycarbonitride, a new composition of matter, can be synthesized by the thermal decomposition of an amine molybdate, in a nonoxidizing and preferably reducing atmosphere. The composition can be obtained possessing catalytic properties and very high surface areas, e.g. above 60 sq metres gm, for instance 60—130 $m^2/g$. (as measured by standard argon-BET methods) and can be pyrophoric at room temperature. The material can readily be passivated for use as a catalyst under ambient conditions.

It has also been unexpectedly found that molybdenum oxycarbonitride is an excellent catalyst for use in CO hydrogenation utilizing mixtures of CO and $H_2$, for selectively producing light paraffinic hydrocarbons in high selectivity over a broad range of operating process variables.

The catalyst in the process exhibits high activity at low $H_2$/CO ratios, which means that the catalyst can operate efficiently on carbon monoxide product streams from gasification of refractory carbon as directly produced. Second, the catalyst shows substantial selectivity for producing light paraffinic hydrocarbons, particularly $C_2$—$C_5$ paraffins, which could be used as a clean storable fuel or as a feedstock for steam cracking. Third, this selectivity for $C_2$—$C_5$ products is maintained over a broad reaction temperature range. This stable product distribution with increasing reaction temperature allows high temperature operations with attendant optimum reaction rates, surprisingly without substantial increases in methane production. Especially noteworthy is the ability of the novel catalyst to selectively produce $C_2$—$C_5$ hydrocarbons under hydrogen-deficient conditions, i.e., greater than 1:3 CO/$H_2$ volume ratios, and to selectively produce this particular hydrocarbon distribution under a wide range of process conditions.

The subject molybdenum oxycarbonitride has also been found to be an excellent catalyst for use in CO hydrogenation utilizing steam for selectively producing light paraffinic hydrocarbons and particularly $C_1$—$C_3$ paraffins and mixtures thereof. This is especially surprising since the composition could be viewed as being particularly reactive with water. Further, it has been observed that no significant carbon deposition occurs on the catalyst during a typical CO hydrogenation with steam.

Furthermore, it has been found that the subject composition, molybdenum oxycarbonitride, is an

effective and highly active catalyst in the synthesis of ammonia from gaseous mixtures of hydrogen and nitrogen. In particular, high rates of ammonia production at one atmosphere can be obtained relative to other processes using commercially available iron-based catalysts.

In accordance with this invention, there is provided a composition comprising molybdenum oxycarbonitride which if poorly or substantially crystalline possesses a face-centered, cubic structure in which the oxygen, carbon and nitrogen atoms are distributed throughout the bulk structure. The composition can be amorphous, poorly crystalline or substantially crystalline, and when crystalline, has the general formula: $MoO_aC_bN_c$, wherein a, b and c are non-zero decimal values, and wherein the sum: a + b + c, is less than or equal to about one.

Also provided is a process for preparing the subject composition comprising the step of thermally decomposing an "amine molybdate" at elevated temperature in the presence of a nonoxidizing atmosphere, wherein said amine contains at least one single bond C—N grouping.

Further, in accordance with this invention, there is provided a process for preparing paraffinic hydrocarbons including linear and branched $C_1$—$C_{10}$ carbon chain numbers comprising contacting a gaseous mixture of CO and $H_2$ in a $CO/H_2$ volume ratio of 10:1 to 1:10, respectively, with molybdenum oxycarbonitride catalyst, described above, at a temperature of about 100 to 600°C, a pressure of about 0.1 to 100 MPa, and a space velocity of about 100 to 50,000 v/v/hr., and recovering product paraffinic hydrocarbons.

In addition, there is provided a process for preparing light paraffinic hydrocarbons including linear and branched $C_1$—$C_{10}$ carbon chain numbers, comprising contacting a gaseous mixture of CO and steam in a CO/steam volume ratio of 10:1 to 1:10, respectively, with molybdenum oxycarbonitride catalyst, described above, at a temperature in the range of about 100 to 600°C, a pressure of about 0.1 to 100 MPa, and a space velocity of about 100 to 50,000 v/v/hr., and recovering product paraffinic hydrocarbons.

Furthermore, there is provided a process for synthesizing ammonia comprising contacting a gaseous mixture of hydrogen and nitrogen in about a 1:3 to 10:1 volume ratio, respectively, with a molybdenum oxycarbonitride catalyst, described above, in a temperature range of about 300 to 500°C, a pressure of about 0.1 to 20 MPa, and a space velocity of about 1000 to 60,000 v/v/hr., thereby resulting in product ammonia.

In the drawings:

Figure 1 depicts comparative X-ray powder diffraction patterns on the same scale of:

(a) pyrophoric molybdenum oxycarbonitride (under Kapton film) obtained by the thermal decomposition of ethylenediammonium molybdate in a helium atmosphere at about 650°C, and

(b) passivated molybdenum oxycarbonitride, obtained by contacting the pyrophoric form at room temperature with an atmosphere of oxygen/helium. Also illustrated are the peak indices based on the standard cubic unit cell.

Figure 2 is a schematic representation of the face-centered cubic molybdenum oxycarbonitride crystal structure, illustrating the metal atom (M) and heteroatom (H) substructures.

Figure 3 depicts a thermogram (A) and its derivative (B) illustrating the decomposition of an ethylene-diamine adduct of molybdic acid to molybdenum oxycarbonitride as obtained by thermogravimetric analysis.

The present composition, molybdenum oxycarbonitride belongs to a general class of materials including interstitial carbides and nitrides which because of their physical nature are often not capable of being precisely defined in terms of unique compositional properties as are for example, organic compounds. Interstitial carbides and nitrides, as a class, are generally comprised of two interpenetrating substructures: a metal atom substructure and a heteroatom substructure. The metal atom substructure dominates X-ray scattering, predominately determines the X-ray diffraction pattern, and forms a polyhedral array within which the heteroatoms occupy certain positions in the polyhedral interstices, for example as depicted in Figure 2. Generally, the metal atom substructure in this class of materials differs from the metal atom arrangement in the pure metal, which is usually evidenced by a difference in the X-ray powder diffraction patterns of the materials. However, usually in both cases there is a periodic ordering of the metal atoms. By contrast, the heteroatom substructure may or may not exhibit a periodic ordering and as a further complicating factor, can in general accommodate a varying number of heteroatom vacancies. Thus, materials in this class of compounds generally exhibit non-stoichiometry with respect to the number of heteroatoms in the interstitial substructure which renders precise definition of the heteroatom substructure very difficult. Further, several different metal atom substructure arrangements are possible for a given empirical formula. Consequently, this class of materials usually exhibits complex phase diagrams, for example, as is known for molybdenum carbide and tungsten carbide. Thus, precise and complete structural characterization of this class of materials is a difficult task since the materials in addition to their non-stoichiometric nature, may also be air-sensitive, pyrophoric, high-melting, and insoluble in standard organic solvents.

With the above discussion as a background, analysis by X-ray diffraction, elemental analysis and thermogravimetric analysis, indicates that the metal atom substructure of crystalline molybdenum oxycarbonitride prepared by the process described herein is reasonably believed to be represented as a face cubic-centered lattice as illustrated in Figure 2. Within the face-centered cubic structure of molybdenum oxycarbonitride as illustrated in Figure 2, it is believed that each heteroatom site is surrounded by six molybdenum atoms while each molybdenum atom, in turn, is surrounded by six heteroatom sites. Thus, the ratio of the number of the heteroatom sites to the number of molybdenum atom is ideally 1:1. Assuming a random distribution for oxygen, carbon and nitrogen atoms throughout the bulk structure, as well as complete occupancy of all heteroatom sites in the heteroatom substructure, leads to the ideal empirical formula for the crystalline composition: $MoO_{1/3}C_{1/3}N_{1/3}$. (Amorphous materials will vary from this formula due to differences in the number of heteroatom sites as compared to the crystalline structure.) However, since the material can probably support a varying number of heteroatom vacancies within the heteroatom substructure as discussed above, and furthermore, since the ratio of O:C:N atoms, which are reasonably believed to be distributed throughout the bulk structure, rather than locally on the surface of the material, during synthesis would not be expected to be incorporated in exactly a 1:1:1 ratio, it follows that elemental analysis indicates only a general overall heteroatom content and that the ratio of O:C:N will vary to a great extent in the composition.

Within this context, the formula of the novel crystalline composition is $MoO_aC_bN_c$, where a, b and c are non-zero decimal values and the sum: a + b + c, due to non-stoichiometry and difficulty in exact measurement, is less than or equal to about one. (The sum of one being the ideal case under the conditions of exact measurement and complete occupany of the heteroatom sites). The ranges for the individual decimal values of a, b and c can vary then as described above with the proviso that each individual value is greater than zero and the sum: a + b + c, is not greater than about one for the pure composition.

A particular example, described herein, is crystalline $MoO_{0.43}C_{0.31}N_{0.33}$, prepared by the decomposition of ethylenediammonium molybdate, whose X-ray diffraction pattern is illustrated in Figure 1.

Molybenum oxycarbonitride is generally pyrophoric when freshly prepared by the process described herein and in order to stabilize the material for use as a catalyst it is usually necessary to passivate it by surface oxidation at room temperature by conventional techniques. For example, the passivating step can be conducted by contacting the pyrophoric material with a stream of oxygen/helium at room temperature in stages of increasing oxygen concentration from about 2 to 10 volume percent or higher, to yield, for example, $MoO_{0.99}C_{0.31}N_{0.33}$, whose X-ray diffraction pattern is illustrated in Figure 1(B). Thus, passivated molybdenum oxycarbonitride is also deemed to be within the scope of the present composition as well as molybdenum oxycarbonitride compositions including other impurities which may also be present.

X-ray diffraction analysis (see Figure 1) also is consistent with the high surface areas of the obtained composition. Surface areas, as measured by the well-known argon-BET method, can be of the order of about 10 to 130 $m^2/g$ and higher and this is evidenced by relatively wide peak half-widths in the X-ray diffraction pattern of Figure 1(A). Here, a crystallite size of about 30—40 Angstroms (3—4 mm) is inferred from the observed half-widths.

The composition can exist in amorphous, poorly crystalline, or crystalline forms which can be evidenced by their respective X-ray diffraction patterns.

By the term "amorphous" is meant an X-ray diffraction pattern exhibiting essentially a straight line. By the term "poorly crystalline" is meant an X-ray diffraction pattern exhibiting distinct yet broad scattering peaks, evidence of a face-centered cubic structure, as depicted in Figure 1-A. By the term "crystalline" is meant an X-ray diffraction pattern exhibiting very sharp scattering peaks, evidencing a face-centered cubic structure. In general, large particle size crystalline materials exhibit narrow diffraction lines and amorphous materials exhibit very broad diffraction lines. Further, it is reasonably believed that the amorphous material when heated or caused to undergo particle size growth, will exhibit the face-centered cubic structure in the final crystalline form.

Molybdenum oxycarbonitride prepared by the process described herein is generally a black powder, having a very high melting point, is extremely insoluble in common organic solvents, is air and water-sensitive, generally has a crystallographic density of about 8.0—10.1 $g/cm^3$, and is usually pyrophoric at room temperatures. The lattice parameters exhibited by poorly crystalline molybdenum oxycarbonitride, prepared by the preferred process is in the range of about 4.1 to 4.4 Angstroms (0.41 to 0.44 nm) and can be influenced by the particular atmopshere used as described below.

Also a subject of this invention is a process for preparing the subject composition. Generally, the process comprises thermally decomposing an "amine molybdate" at elevated temperature, under a non-oxidizing atmosphere, preferably a reducing atmosphere, wherein said amine contains at least one single bond C—N grouping, and recovering the resulting molybdenum oxycarbonitride.

By the term "molybdate" as used herein is meant molybdates, polyoxomolybdates, including those formed from molybdic acids, oxides and acid anhydrides, of the formulas: $MoO_3.xH_2O$, $H_2MoO_4$, $H_2Mo_2O_7$, $H_2Mo_3O_{10}$, $H_6Mo_7O_{24}$, $H_4Mo_8O_{26}$ and the like.

By the term "amine molybdate" as used herein, is meant a compound, salt, complex or adduct formed between the interaction of an amine and respective molybdic acid, oxide or anhydride as described herein.

Amines that are operable in this invention are alkyl amines, alkylenediamines and aromatic amines that preferably contain 1—20 carbon atoms per molecule and contain at least one single bond C—N grouping. The reason why this structural limitation is necessary in the process is not clear. One

theory that we do not wish to be bound by is that the single bond C—N grouping enables both the carbon and nitrogen fragments of the amine to be simultaneously incorporated upon decomposition into the final oxycarbonitride. Thus, pyridine and ammonia are inoperable as the amine components of the amine molybdate in the process, whereas 4-ethyl-aminopyridine and ethylamine are operable amine components.

The alkylamines and alkylenediamines may be linear or branched and may also contain substituents which are inert under the reaction conditions, e.g., alkoxy or halogen. The aromatic amines may also contain substituents on the aromatic ring which are inert under the reaction conditions, e.g., alkoxy or halogen.

Representative examples include ethylenediamine, butylamine, ethylamine, diethylamine, di-n-butylamine, trimethylamine, triethylamine, 1,3-cyclohexane bismethylamine, aniline, 4-ethylaminopyridine and the like and mixtures thereof. A preferred amine in the process is ethylene-diamine.

The amine molybdate is treated in the process by subjecting the compound to a temperature sufficient to cause thermal decomposition. By the term "thermal decomposition" is meant the process of thermal rearrangement of the compound involving usually carbon-carbon, carbon-nitrogen, molybdenum-oxygen and also carbon-hydrogen bond breaking, thereby resulting in molybdenum oxycarbonitride. Temperatures at which thermal decomposition of the operable amine molybdate occurs depends upon the particular amine molybdate employed and is usually in the range of about 150 to 800°C, preferably about 300 to 700°C, and particularly preferred about 500 to 600°C.

The thermal decomposition is conducted under a non-oxidizing atmosphere, which can be inert and/or reducing in nature, preferably reducing, and includes hydrogen, carbon monoxide, helium and the like, and mixtures thereof. In the process there is preferably a substantial absence of elemental oxygen and water at temperatures above 150°C during thermal decomposition.

It has been found that in the case of ethylenediammonium molybdate, the compound can be thermally decomposed if desired in an atmosphere substantially comprising helium, exclusively. However, with other amine molybdates an atmosphere containing some hydrogen gas is usually necessary. Preferably, a small amount of hydrogen gas is used to counteract small traces of oxygen or moisture which may be present in the process atmosphere. The process atmosphere preferably contains about 25—50 volume % $H_2$ in admixture with helium or carbon dioxide.

The process atmosphere can be maintained at atmospheric pressure, under reduced pressure, or greater than atmospheric pressure. Preferably the thermal decomposition is conducted at atmospheric pressure. The process atmosphere can be continuous and dynamic as in a constant flowing stream or used in a tube furnace or can be a static atmosphere as present in an autoclave. Preferred is a constant flowing stream of the atmospheric gaseous mixture.

Space velocity of the inert/reducing gas in a flowing, dynamic atmosphere is not critical and can be conveniently conducted in the range of about 100 to 50,000 v/v/hr. What is important is that the flow of gas should be sufficient to sweep away gaseous by-products from the reaction zone and to maintain a sufficiently high concentration of reducing atmosphere in the vicinity of the amine molybdate.

Apparatus for carrying out the thermal decomposition may be any conventional type known to those skilled in the art and include stainless steel and glass tube furnaces, autoclaves, and the like.

The subject molybdenum oxycarbonitride composition is also useful in producing hydrocarbons by catalyzing the reaction between CO and $H_2$ at elevated temperature.

The process is conducted by contacting a mixture of carbon monoxide and hydrogen with the above-described catalyst in a conventional reactor. Representative types of reactors and apparatus that can be employed are glass and stainless steel reactors that are vertical, horizontal or down-flow types which utilize the catalyst as a fixed bed, fluid bed, slurry and the like.

Generally, the catalyst is initially used in the passivated form, which is relatively stable, to avoid decomposition. Then the catalyst is generally pretreated at an elevated temperature in a reducing atmosphere for a period of time to generate the reduced form of the catalyst prior to the process. The temperature, atmosphere and time required are conventional in the art. A set of conditions which was found to be effective was pretreatment at 450°C in a hydrogen atmosphere, at a space velocity of about 10,000 v/v/hr. for a time of about 2 hours. Other sets of conditions will be obvious to one skilled in the art.

The gaseous mixture of CO and $H_2$ is used in a $CO/H_2$ volume ratio of 10:1 to 1:10, respectively, and preferably a 1:3 to 2:1 ratio. Particularly preferred is a 1:1 or $CO/H_2$ volume ratio, for example as obtained directly from a coal gasification process.

The CO and hydrogen gases used in the process can also be commercially available, for example, reagent, technical or industrial purity and can contain small amounts of other gases which are inert under the reaction conditions such as nitrogen, argon or helium, which can also be used as carrier gases in the process.

The temperature of the process is generally conducted in the range of about 100° to 600°C, and preferably about 225—450°C.

The pressure of the $CO/H_2$ feedstream in the process is generally carried out in the region of about 0.1 to 100 MPa and preferably about 0.1 to 3.0 MPa (1 atmosphere being equivalent to

0.1 MPa).

The space velocity of the $CO/H_2$ feedstream (including carrier gas, if used) in the process is generally carried out in the range of about 100 to 50,000 v/v/hr. and preferably about 1000 to 5000 v/v/hr.

Product hydrocarbons are collected, separated and purified by conventional methods in the art.

The product paraffinic hydrocarbons include linear and branched $C_1$—$C_{10}$ hydrocarbons, preferably being linear, and include methane, ethane, propane, butane, pentane, hexane, heptane, octane, nonane, decane, isooctane, neodecane, isopentane, neopentane and the like. Preferred are $C_2$—$C_5$ in carbon number, being ethane, propane, butane and pentane, and mixtures thereof, as the main products in the process. Methane is also substantially produced in the process.

Yields in the process of paraffinic hydrocarbons can also vary depending upon the exact conditions employed. Yields of combined $C_2$—$C_5$ hydrocarbons can be about 30—70 wt.% of total hydrocarbons and higher. Yields of methane can be about 30—70 wt.% of total hydrocarbon produced also. By the term "mainly comprised" as used herein is meant that about 30—70 wt.% of total hydrocarbons produced are comprised of ethane, propane, butane, pentane, and mixtures thereof.

Percent CO conversions can vary in the process, depending upon the specific conditions employed, and can be in the range of about 20—80% and higher. Higher pressures generally lead to greater % CO conversions.

The subject CO/steam invention process is conducted by contacting a mixture of carbon monoxide and steam with the above-described catalyst in a conventional reactor. Representative types of reactors and apparatus that can be employed are glass and stainless steel reactors that are vertical, horizontal or down-flow types which utilize the catalyst as a fixed bed, fluid bed, slurry and the like.

The catalyst is generally pretreated at an elevated temperature in a reducing atmosphere for a period of time prior to the process. The temperature, atmosphere and time required are conventional in the art. A set of conditions which was found to be effective was pretreatment at 450°C in a hydrogen atmosphere, at a space velocity of about 10,000 v/v/hr. for a time of about 2 hours. Other sets of conditions will be obvious to one skilled in the art.

The gaseous mixture of CO and steam is in a CO/steam volume ratio of 10:1 to 1:10, respectively, and preferably in a 1:3 to 3:1 ratio.

The CO used in the process can be commercially available, of high purity and can contain small amounts of other gases which are inert under the reaction conditions such as nitrogen which can also be used as a carrier gas.

Steam for use in the process can be generated, for example, by saturating the CO feedstream with a conventional steam saturator.

The temperature of the process is conducted in the range of about 100 to 600°C, and preferably about 225—450°C.

The pressure of the CO/steam feedstream in the process is carried out in the region of about 0.1 to 100 MPa and preferably about 0.1 to 3.0 MPa (1 atmosphere being equivalent to 0.1 MPa).

The space velocity of the CO/steam feedstream in the process is carried out in the range of 100 to 50,000 v/v/hr. and preferably about 100 to 2500 v/v/hr.

Product hydrocarbons are collected, separated and purified by conventional methods in the art.

The product paraffinic hydrocarbons include linear and branched $C_1$—$C_{10}$ hydrocarbons, preferably linear and include methane, ethane, propane, butane, pentane, hexane, heptane, octane, nonane, decane, isooctane, neodecane, isopentane, neopentane and the like. Preferably, $C_1$—$C_3$ hydrocarbons including methane, ethane, propane, and mixtures thereof, are preferred products in the process.

Percent CO conversions can vary in the process, depending upon the specific conditions employed, and can be in the range of about 30—80% and higher.

The subject ammonia process is conducted by contacting a mixture of hydrogen and nitrogen gas with the above-described molybdenum oxycarbonitride catalyst under the following conditions described herein and collecting product ammonia.

The gaseous mixture of hydrogen/nitrogen used is in the volume ratio of about 1:3 to 10:1 and preferably the ratio is about 3:1.

The temperature of the process is conducted in the range of about 300 to 500°C and preferably about 350 to 450°C.

The pressure of the gaseous feedstream in the process is carried out in the region of about 0.1 to 20 MPa and preferably 2 to 15 MPa (1 atmosphere being equivalent to 0.1 MPa).

The space velocity of the process is carried out in the range of about 1000 to 60,000 v/v/hr. and preferably about 5000 to 50,000 v/v/hr.

Product ammonia in the process is collected, separated and purified by conventional methods in the art.

The following examples are illustrative of the best mode of carrying out the invention as contemplated by us.

Example 1

Preparation of Ethylenediammonium Molybdate

50 g of molybdic acid, $H_2MoO_4$, and 750 ml of pure ethylenediamine were refluxed under stirring for about 16 hours. The resulting white-colored solid was collected on a fritted funnel washed with ethanol to remove any unreacted ethylenediamine and then dried in an oven to remove the ethanol. Obtained was a white solid having the following elemental analysis: %N, calculated 12.6, found 13.99; %Mo, calculated 43.2, found 46.9. The above-described preparation was found to be reproducible.

Thermal Decomposition

Thermal decomposition of the above-described ethylenediammonium molybdate was carried out by preheating a glass tube furnace at about 650°C, through which a stream of helium was flowing at 600 ml/min. The above-prepared sample was placed into a quartz boat which was then placed into the tube furnace. The temperature was allowed to equilibrate for about 5 minutes and then the sample was heated to 650°C, under the helium atmosphere, for about 20 minutes to effect the decomposition. Subsequently, the tube was allowed to cool to room temperature, and the tube was then flushed with helium gas for about 15 minutes. Obtained was a black, pyrophoric material which had the approximate composition: $MoO_{0.43}C_{0.31}N_{0.33}$. The pyrophoric material was passivated by contacting the solid at room temperature with a gaseous mixture of oxygen and helium containing increasing concentrations of oxygen according to the follwing schedule:

| Feedstream | Time |
|---|---|
| 2% $O_2$/He | 1 hour |
| 4% $O_2$/He | 1 hour |
| 6% $O_2$/He | 1 hour |
| 10% $O_2$/He | 1 hour |

The resulting composition had the empirical formula: $MoO_{0.99}C_{0.31}N_{0.33}$ based on results of the chemical analysis: Mo, 79.77%; O, 13.16%; C, 3.05%; N, 3.79%; H, 0.42%. X-ray analysis showed that in the pyrophoric material, the molybdenum atoms form a cubic closest packed array. Thus, the empirical formula $MoO_{0.43}C_{0.31}N_{0.33}$, indicates that all octahedral interstices are occupied by O, C and N atoms, respectively (0.43 + 0.31 + 0.33 = 1.07). X-ray line broadening techniques revealed that the passivated material had an average particle size of about 30 Angstroms (Å) (3 nm).

Carrying out the thermal decomposition in carbon monoxide, rather than helium, at 650°C, resulted in a material exhibiting the same face-centered cubic lattice, but having a significantly larger lattice constant, being 4.21 Å versus 4.13 Å. The largest lattice constant, 4.32 Å, was obtained using a reducing atmosphere comprising helium/carbon monoxide mixture.

Example 2

The following described experiments were conducted to illustrate the reversibility of the passivated and surface active crystalline forms of the above-described oxycarbonitride.

*A*. The passivated material described in Example 1 was treated at 450°C with a stream of hydrogen for 0.5 hour. The resulting pyrophoric black solid had a surface area of about 135 $m^2/g$ (as measured by standard-argon BET method).

*B*. The resulting material from *A* above was passivated by the procedure described in Example 1 resulting in a passivated material having an argon BET surface area of about 98 $m^2/g$.

*C*. The resulting material from *B* above was treated with a stream of $H_2$ at 450°C for 0.5 hours resulting in a pyrophoric black powder having a BET surface area of about 131 $m^2/g$.

Example 3

Ethylenediammonium molybdate prepared as in Example 1 was thermally decomposed in the tube furnace described in Example 1, at 350°C with a stream of helium (600 ml/min.) for 1.5 hours. The resulting black solid had an argon-BET surface area of about 18 $m^2/g$ and the X-ray diffraction pattern indicated the solid was "amorphous" molybdenum oxycarbonitride.

Example 4

Following the general procedure outline in Example 1, the following amine molybdates, prepared from molybdic acid ($H_2MoO_4$), were thermally decomposed to yield molybdenum oxycarbonitride. Exact conditions used are listed in Table I. Each of the materials was passivated after formation according to the procedure described in Example 1.

TABLE I

| Amine Molybdate | Temp. | Atmosphere[a] | Time |
|---|---|---|---|
| (1) 1,3-cyclohexane-bis-methylamine | 400°C | 75/25 $H_2$/He | 20 min. |
| (2) trimethylamine | 400°C | 100% $H_2$ | 20 min. |
| (3) di-n-butylamine | 400°C | 75/25 $H_2$He | 20 min. |
| (4) aniline | 400°C | 75/25 $H_2$/He | 20 min. |
| (5) dimethylamine | 400°C | 50/50 $H_2$/He | 20 min. |
| (6) ethylamine | 400°C | 50/50 $H_2$/He | 20 min. |

[a] flow rate of 600 ml/min.

X-ray diffraction analyses indicated that molybdenum oxycarbonitride was formed in each of the above cases.

Comparative Example 1

Ammonium molybdate (commercially available) was thermally treated following the general procedure described in Example 1 in a 1:1 $CO/H_2$ flowing atmosphere at 500°C. Analysis by X-ray diffraction of the obtained solid indicated that it was not molybdenum oxycarbonitride.

Comparative Example 2

A pyridine salt of molybdic acid (prepared by refluxing a mixture of molybdic acid and pyridine) was thermally treated in an argon atmosphere at 400°C according to the general procedure described in Example 1. Analysis by X-ray diffraction of the obtained solid indicated that it was not molybdenum oxycarbonitride.

Example 5

Five-tenths cc of passivated molybdenum oxycarbonitride was placed in a down flow pyrex glass reactor. The catalyst, as a fixed bed, was pretreated in situ with hydrogen gas at 450°C for 1 hour at 0.1 MPa and at a space velocity of about 24,000 v/v/hr. Four runs were conducted by passing a stream of $CO/H_2$ in varying volume ratios of from 1:3 to 4:1 at a space velocity of 2,400 v/v/hr. and a pressure of 0.1 MPa, over the catalyst maintained at a temperature of about 299°C. The products from each run were analyzed by on-line gas chromatography. Results are indicated below in Table II.

TABLE II

| $CO/H_2$ Ratio | Temp. | %CO Conv.[a] | Rate[b] | Hydrocarbon Products[c] $C_1$ | $C_2$ | $C_3$ | $C_4$ | $C_5$+ |
|---|---|---|---|---|---|---|---|---|
| 1:3 | 299°C | 34.2 | 149.2 | 54 | 31 | 11 | 3 | 1 |
| 1:1 | 299°C | 17.3 | 150.9 | 43 | 34 | 17 | 4 | 2 |
| 2:1 | 299°C | 8.4 | 73.3 | 41 | 37 | 17 | 4 | 1 |
| 4:1 | 299°C | 2.2 | 38.4 | 44 | 42 | 13 | t[d] | t[d] |

[a] Percent CO conversion;
[b] Rate of CO conversion in micromoles CO/min./g of catalyst;
[c] Wt.% of total hydrocarbons produced;
[d] Trace quantity

As is seen from the above Table, equivalent rates of CO conversion are observed at $CO/H_2$ reactant ratios of 1:3 to 1:1. The rate decreased when the $CO/H_2$ ratio was increased above 1:1. The $CO/H_2$ volume ratio had little effect on the overall hydrocarbon product distribution. Reducing the $CO/H_2$ volume ratio brought only a small decrease in methane and small increase in higher hydrocarbon selectivities, demonstrating the stable product composition with changing reactant gas composition.

Example 6

Using the same catalyst, apparatus and general procedure described above in Example 5, a series of runs was conducted to illustrate the influence of temperature on the hydrocarbon product selectivity. Five runs were made at temperatures ranging from 254° to 315°C, at a $CO/H_2$ volume ratio of 1:1, a pressure of 0.1 MPa, and a space velocity of 2,400 v/v/hr. The results are tabulated below in Table III.

TABLE III

| Temp. (°C) | $CO/H_2$[b] | %CO Conv. | Hydrocarbon Products[a] | | | | |
|---|---|---|---|---|---|---|---|
| | | | $C_1$ | $C_2$ | $C_3$ | $C_4$ | $C_5$+ |
| 315 | 1 | 20.0 | 45 | 34 | 16 | 4 | 1 |
| 299 | 1 | 17.3 | 43 | 34 | 17 | 4 | 2 |
| 282 | 1 | 13.1 | 41 | 34 | 18 | 5 | 2 |
| 265 | 1 | 10.1 | 39 | 33 | 21 | 5 | 2 |
| 254 | 1 | 8.1 | 39 | 33 | 20 | 6 | 2 |

[a] Wt. percent of total hydrocarbons produced
[b] Volume ratio

As is seen, decreasing the process temperature brought about a slight decrease in selectivity to methane. However, the product distribution was not substantially affected by changes in reaction temperature, demonstrating the stability of the product distribution over a wide range of operating temperatures.

Example 7

Five (5) cc of passivated molybdenum oxycarbonitride (prepared by the thermal decomposition of ethylenediammonium molybdate at 450°C and having a BET-argon surface area of about 100 $m^2/g$ were placed into a 1 cm diameter stainless steel down-flow reactor. A thermocouple was placed into the center of the catalyst bed. The catalyst was pretreated in situ by passing a stream of hydrogen over the catalyst at 1 atmosphere, a space velocity of 480 v/v/hr. and a temperature of 400°C for 78 hours. The temperature was then reduced to 300°C and the pressure increased to 120 psia (about 0.8 MPa). The hydrogen feed was replaced by a mixture of 3:1 parts by volume of CO/steam and a small amount of nitrogen as an internal standard. The steam was generated by saturating a stream of carbon monoxide by bubbling through a saturator containing distilled deionized water at 121°C at which the vapor pressure of water was 30 psi. Feedstream lines to the reactor were maintained above 121°C to prevent condensation. The composition of the effluent gases was monitored for a period of 7 hours during the run which was conducted at 300°C, 120 psia (about 0.8 MPa), a space velocity of 400 v/v/hr., and a feed composition of 100 CO:33 $H_2O$ (steam): 2.04 $N_2$. The monitoring process was conducted by passing the reaction mixture through a cold trap maintained at −1°C. The non-condensed gases were analyzed by on-stream gas chromatography versus known standards. Results are listed below in Table IV.

TABLE IV

| Gaseous Products[a] | | |
|---|---|---|
| Methane | — | 21.35 g/$NM^3$ of CO fed |
| Ethane | — | 13.24 g/$NM^3$ of CO fed |
| Propane | — | 15.29 g/$NM^3$ of CO fed |
| Butane | — | 8.94 g/$NM^3$ of CO fed |
| Pentane | — | 5.37 g/$NM^3$ of CO fed |
| Hexane | — | 4.81 g/$NM^3$ of CO fed |
| Carbon Dioxide | — | 716.9 g/$NM^3$ of CO fed |
| Hydrogen | — | 9.45 g/$NM^3$ of CO fed |

[a] CO conversion — 46.9%

No carbon deposition on the catalyst was observed. The weight of the recovered catalyst was substantially the same as the starting catalyst.

Example 8

A 1.0 gram sample of passivated molybdenum oxycarbonitride (of approximate empirical formula: $MoO_{0.41}C_{0.31}N_{0.33}$ and BET-argon surface of about 130 $m^2/g$.) was reduced as a fixed bed catalyst in a 3:1 mixture of $H_2/N_2$ under 0.1 MPa of pressure for a period of about four hours in a glass reactor system. The run was carried out at three different temperatures of about 450°C, 400°C and then about 350°C. The gas hourly space velocity was run at five different rates of 1200, 2400, 3600, 4800 and 6000 v/v/hr. for each temperature used. The rate of ammonia production and the volume percent ammonia in the product stream, as determined by gas chromatography and wet chemical methods are also given below in Table V.

TABLE V
Ammonia Synthesis Activity of Molybdenum Oxycarbonitride

| Temp. °C | Pressure (MPa) | GHSV (v/v/hr) | Rate[a] (micromoles/ min/g) | %$NH_3$[b] |
|---|---|---|---|---|
| 450 | 0.11 | 1200 | 1.77 | 0.198 |
| 450 | 0.11 | 2400 | 3.30 | 0.185 |
| 450 | 0.11 | 3600 | 4.63 | 0.173 |
| 450 | 0.11 | 4800 | 6.00 | 0.168 |
| 450 | 0.11 | 6000 | 7.44 | 0.167 |
| 400 | 0.11 | 1200 | 1.72 | 0.193 |
| 400 | 0.11 | 2400 | 2.80 | 0.157 |
| 400 | 0.11 | 3600 | 3.68 | 0.137 |
| 400 | 0.11 | 4800 | 4.04 | 0.113 |
| 400 | 0.11 | 6000 | 4.38 | 0.098 |
| 350 | 0.11 | 1200 | 0.72 | 0.081 |
| 350 | 0.11 | 2400 | 1.17 | 0.066 |
| 350 | 0.11 | 3600 | 1.46 | 0.055 |
| 350 | 0.11 | 4800 | 1.73 | 0.049 |
| 350 | 0.11 | 6000 | 2.14 | 0.048 |

[a] rate of $NH_3$ production
[b] volume percent $NH_3$ in product gaseous stream

As is seen from the data, appreciable rates of $NH_3$ formation can be obtained over a fairly broad combination of process temperatures and space velocities.

Example 9

A 2.0 gram sample of passivated molybdenum oxycarbonitride (from the same stock of material as described in Example 8) was reduced in a 3:1 volume mixture of $H_2/N_2$ at a gas hourly space velocity of 2400 v/v/hr. for a period of four hours in a stainless steel reactor. The temperature was run at three values: 450°C, 400°C and 350°C and the pressure was adjusted to 0.1, 1.0 and 2.0 MPa for each temperature run. Results are given below in Table VI.

TABLE VI
Ammonia Synthesis Activity of Molybdenum Oxycarbonitride

| Temp. °C | Pressure (MPa) | GHSV (v/v/hr) | Rate[a] (micromoles/ min/g) | $\%NH_3$[b] |
|---|---|---|---|---|
| 450 | 0.11 | 2400 | 2.95 | 0.18 |
| 40 | 1.00 | 2400 | 11.85 | 0.73 |
| 450 | 2.00 | 2400 | 17.85 | 1.16 |
| 400 | 0.11 | 2400 | 2.10 | 0.13 |
| 400 | 1.03 | 2400 | 7.20 | 0.44 |
| 400 | 2.04 | 2400 | 9.35 | 0.58 |
| 350 | 0.11 | 2400 | 1.25 | 0.08 |
| 350 | 1.01 | 2400 | 3.90 | 0.24 |
| 350 | 2.02 | 2400 | 5.45 | 0.34 |

[a] rate of $NH_3$ production
[b] volume percent $NH_3$ in product gaseous stream

As seen from the data, increasing pressure in the process significantly increases the rate of $NH_3$ production at constant space velocity.

**Claims**

1. A composition consisting of molybdenum oxycarbonitride possessing a face-centred cubic structure in which the interstitial oxygen, carbon and nitrogen atoms are distributed throughout the bulk structure.

2. A composition according to claim 1 having the formula: $Mo\ O_a\ C_b\ N_c$ where a, b and c are non-zero decimal values, and wherein the sum: a + b + c, is less than or equal to about 1.

3. A composition according to claim 1 having the formula: $MoO_{0.43}C_{0.31}N_{0.33}$.

4. An amorphous composition consisting of molybdenum oxycarbonitride in which the interstitial oxygen, carbon and nitrogen atoms are distributed throughout the bulk structure.

5. A composition according to any one of claims 1 to 3 which is of poorly crystalline structure.

6. A composition according to any one of the preceding claims possessing an argon-BET surface area of at least 60 square meters per gram.

7. A composition according to any one of the preceding claims which is passivated with oxygen.

8. A composition consisting of molybdenum oxycarbonitride obtainable by a process comprising decomposing thermally as "amine molybdate" at an elevated temperature under a non-oxidising atmosphere and recovering the resulting molybdenum oxycarbonitride, said amine molybdate being a compound, salt, complex or adduct formed between the interaction of an amine containing at least one single bond C—N grouping and the respective molybdic acid, oxide or anhydride of the formulae:

$$MoO_3.xH_2O, H_2MoO_4, H_2Mo_2O_7, H_2Mo_3O_{10}, H_6Mo_7O_{24} \text{ or } H_4Mo_8O_{26}.$$

9. A process for producing the composition according to any one of the preceding claims which comprises thermally decomposing an "amine molybdate" at elevated temperature in the presence of a nonoxidising atmosphere wherein said amine contains at least one single bond C—N grouping.

10. A process for preparing paraffinic hydrocarbons including linear and branched $C_1$—$C_{10}$ carbon chain numbers comprising contacting a gaseous mixture of CO and $H_2$ in a $CO/H_2$ volume ratio of 10:1 to 1:10, respectively, with the composition claimed in any of claims 1 to 8, at a temperature of about 100 to 600°C, a pressure of about 0.1 to 100 MPa, and a space velocity of about 100 to 50,000 v/v/hr., and recovering product paraffinic hydrocarbons.

11. A process for preparing paraffinic hydrocarbons including linear and branched $C_1$—$C_{10}$ carbon chain number comprising contacting a gaseous mixture of CO and steam in a CO/steam volume ratio of 10:1 to 1:10, respectively, with the composition according to any one of claims 1 to 8, at a temperature in the range of about 100 to 600°C, a pressure of about 0.1 to 100 MPa, and a space velocity of

about 100 to 50,000 v/v/hr., and recovering product paraffinic hydrocarbons.

12. A process for synthesising ammonia comprising contacting a gaseous mixture of hydrogen and nitrogen, in a 1:3 to 10:1 volume ratio, respectively, with the composition according to any one of claims 1 to 8, at a temperature in the range of about 300 to 500°C, a pressure of about 0.1 to 20 MPa, and a space velocity of about 1000 to 60,000 v/v/hr., thereby resulting in product ammonia.

**Revendications**

1. Composition constituée d'un oxycarbonitrure de molybdène ayant une structure cubique à face centrée dans laquelle les atomes d'azote, de carbone et d'oxygène interstitiels sont répartis dans la masse.

2. Composition selon la revendication 1 de formule: Mo $O_a$ $C_b$ $N_c$, dans laquelle a, b, et c sont des valeurs decimales différentes de zéro et dans laquelle la somme a + b + c est inférieure ou égale à 1 environ.

3. Composition selon la revendication 1 de formule: $MoO_{0,43}C_{0,31}N_{0,33}$.

4. Composition amorphe constituée d'un oxycarbonitrure de molybdène dans lequel les atomes d'azote, de carbone et d'oxygène sont répartis dans la masse.

5. Composition selon l'une quelconque des revendications 1 à 3, qui a une structure faiblement cristalline.

6. Composition selon l'une quelconque des revendications précédentes ayant une surface BET d'au moins 60 $m^2/g$.

7. Composition selon l'une quelconque des revendications précédentes passivée à l'oxygène.

8. Composition constituée d'un oxycarbonitrure de molybdène obtenu par un procédé qui consiste à décomposer thermiquement un molybdate d'amine à température élevée sous atmosphère non oxydante et à isoler l'oxycarbonitrure de molybdène résultant, ledit molybdate d'amine étant un composé, sel, complexe ou adduit formé par action d'une amine contenant au moins un groupe à liaison simple C—N sur l'acide, l'oxyde ou l'anhydride molybdique de formule respective:

$$MoO_3,xH_2O,H_2MoO_4,H_2Mo_2O_7,H_2Mo_3O_{10},H_6Mo_7O_{24} \text{ ou } H_4Mo_8O_{26}.$$

9. Procédé de préparation d'une composition selon l'une quelconque des revendications précédentes qui consiste à décomposer thermiquement un molybdate d'amine à température élevée en atmosphère non oxydante dans lequel ladite amine contient au moins un groupe à liaison simple C—N.

10. Procédé de préparation d'hydrocarbures paraffiniques comprenant des chaînes linéaires ou ramifiées de $C_1$ à $C_{10}$ qui consiste à mettre en contact un mélange gazeux de CO et $H_2$, le rapport des volumes de $CO/H_2$ étant compris entre 10:1 et 1:10, respectivement avec une composition revendiquée dans l'une quelconque des revendications 1 à 8, à une température de 100 à 600°C environ, une pression de 0,1 à 100 MPa, et une vitesse de 100 à 50000 V/V/h, et à isoler les hydrocarbures paraffiniques formés.

11. Procédé de préparation d'hydrocarbures paraffiniques comprenant des chaînes droites ou linéaires en $C_1$ à $C_{10}$ qui consiste à mettre en contact un mélange gazeux de CO et de vapeur d'eau, le rapport des volumes de CO/vapeur étant compris entre 10:1 et 1:10, respectivement, avec une composition selon l'une quelconque des revendications 1 à 8, à une température comprise entre 100 et 600°C environ, une pression de 0,1 à 100 MPa et une vitesse de circulation de 100 à 50000 V/V/h environ, et à isoler les hydrocarbures paraffiniques obtenus.

12. Procédé de synthèse de l'ammoniaque qui consiste à mettre en contact un mélange d'hydrogène et d'azote, le rapport des volumes étant de 1:3 à 10:1, respectivement, avec une composition selon l'une quelconque des revendications 1 à 8, à une température comprise entre 300 et 500°C environ, une pression de 0,1 à 20 MPa et une vitesse de circulation de 1000 à 60000 V/V/h, ce qui entraîne la formation d'ammoniac.

**Patentansprüche**

1. Zusammensetzung bestehend aus Molybdänoxycarbonitrid mit einer flächenzentrierten kubischen Struktur, in der die Zwischengittersauerstoff-, -kohlenstoff- und -stickstoffatome über die Gesamtstruktur verteilt sind.

2. Zusammensetzung nach Anspruch 1 mit der Formel Mo $O_a$ $C_b$ $N_c$, in der a, b und c von O abweichende Dezimalwerte sind und die Summe von a + b + c weniger als oder gleich etwa 1 ist.

3. Zusammensetzung nach Anspruch 1 mit der Formel $MoO_{0,43}C_{0,31}N_{0,33}$.

4. Amorphe Zusammensetzung bestehend aus Molybdänoxycarbonitrid, bei dem die Zwischengittersauerstoff, -kohlenstoff- und -stickstoffatome über die Gesamtstruktur verteilt sind.

5. Zusammensetzung nach einem der Ansprüche 1 bis 3, dadurch gekennzeichnet, daß sie eine schwach ausgeprägte kristalline Struktur besitzt.

6. Zusammensetzung nach einem der vorangehenden Ansprüche, dadurch gekennzeichnet, daß sie eine Argon-BET-Oberfläche von mindestens 60 $m^2/g$ besitzt.

7. Zusammensetzung nach einem der vorangehenden Ansprüche, dadurch gekennzeichnet, daß sie mit Sauerstoff passiviert ist.

8. Zusammensetzung bestehend aus Molybdänoxycarbonitrid erhältlich durch ein Verfahren, bei dem ein Aminmolybdat bei einer erhöhten Temperatur unter einer nicht oxydierenden Atmosphäre thermisch zersetzt wird und das resultierende Molybdänoxycarbonitrid gewonnen wird, wobei das Aminmolybdat eine Verbindung, eine Salz, ein Komplex oder ein Addukt ist, das bzw. der durch Wechselwirkung von einem mindestens eine Einfachbindung C—N enthaltenden Amin und der bzw. dem entsprechenden Molybdänsäure, -oxid oder -anhydrid mit den Formeln

$$MoO_3 . xH_2O, H_2MoO_4, H_2Mo_2O_7, H_2Mo_3O_{10}, H_6Mo_7O_{24} \text{ oder } H_4Mo_8O_{26}$$

gebildet worden ist.

9. Verfahren zur Herstellung der Zusammensetzung nach einem der vorangehenden Ansprüche, dadurch gekennzeichnet, daß ein Aminmolybdat bei erhöhter Temperatur in Gegenwart einer nicht oxydierenden Atmosphäre thermisch zersetzt wird, wobei das Amin mindestens eine Einfachbindung C—N enthält.

10. Verfahren zur Herstellung paraffinischer Kohlenwasserstoffe einschließlich linearer und verzweigter $C_1$—$C_{10}$-Kohlenstoffkettenzahlen, dadurch gekennzeichnet, daß eine gasförmige Mischung aus CO und $H_2$ in einem $CO/H_2$-Volumenverhältnis von 10:1 bis 1:10 mit der Zusammensetzung gemäß einem der Ansprüche 1 bis 8 bei einer Temperatur von etwa 100 bis 600°C, einem Druck von etwa 0,1 bis 100 MPa und einem Durchsatz von etwa 100 bis 50 000 V/V/h kontaktiert wird und paraffinische Kohlenwasserstoffe als Produkt gewonnen werden.

11. Verfahren zur Herstellung paraffinischer Kohlenwasserstoffe einschließlich linearer und verzweiger $C_1$—$C_{10}$-Kohlenstoffkettenzahlen, dadurch gekennzeichnet, daß eine gasförmige Mischung aus CO und Dampf in einem CO/Dampf-Volumenverhältnis von 10:1 bis 1:10 mit der Zusammensetzung gemäß einem der Ansprüche 1 bis 8 bei einer Temperatur im Bereich von 100 bis 600°C, einem Druck von etwa 0,1 bis 100 MPa und einem Durchsatz von etwa 100 bis 50 000 V/V/h kontaktiert wird und paraffinische Kohlenwasserstoffe als Produkt gewonnen werden.

12. Verfahren zur Synthese von Ammoniak, dadurch gekennzeichnet, daß eine gasförmige Mischung aus Wasserstoff und Stickstoff in einem Volumenverhältnis von 1:3 bis 10:1 mit der Zusammensetzung gemäß einem der Ansprüche 1 bis 8 bei einer Temperatur im Bereich von etwa 300 bis 500°C, einem Druck von etwa 0,1 bis 20 MPa und einem Durchsatz von etwa 1000 bis 60 000 V/V/h kontakiert wird und zu Ammoniak als Produkt führt.

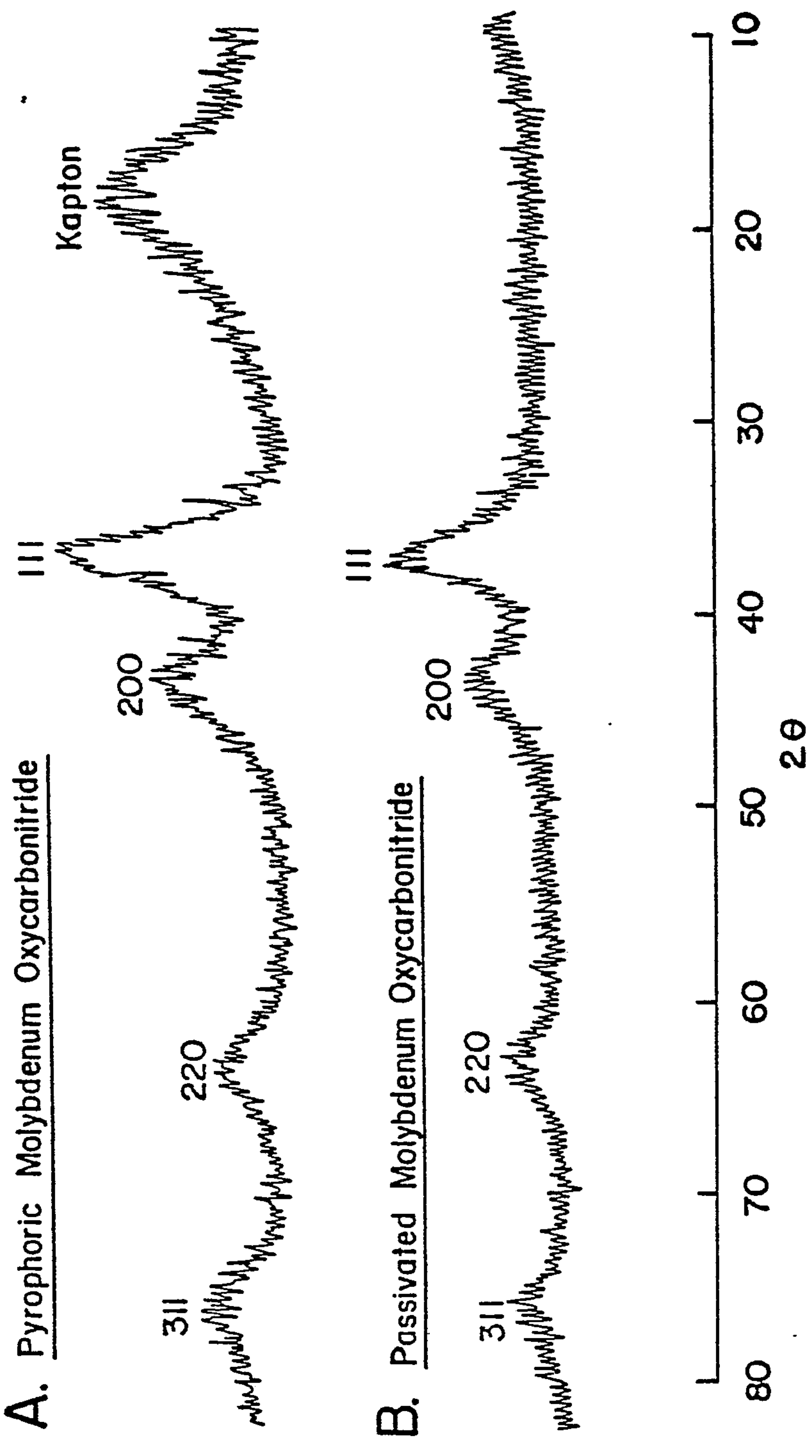
A. Pyrophoric Molybdenum Oxycarbonitride
B. Passivated Molybdenum Oxycarbonitride
Kapton
111
200
220
311
111
200
220
311
10
20
30
40
50
60
70
80
2θ

FIG.1

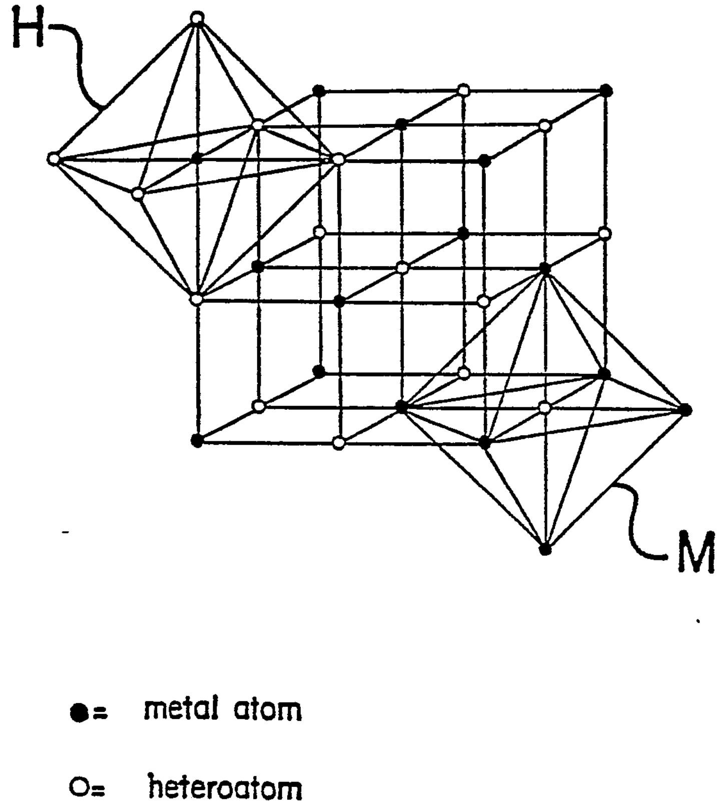


FIG. 2

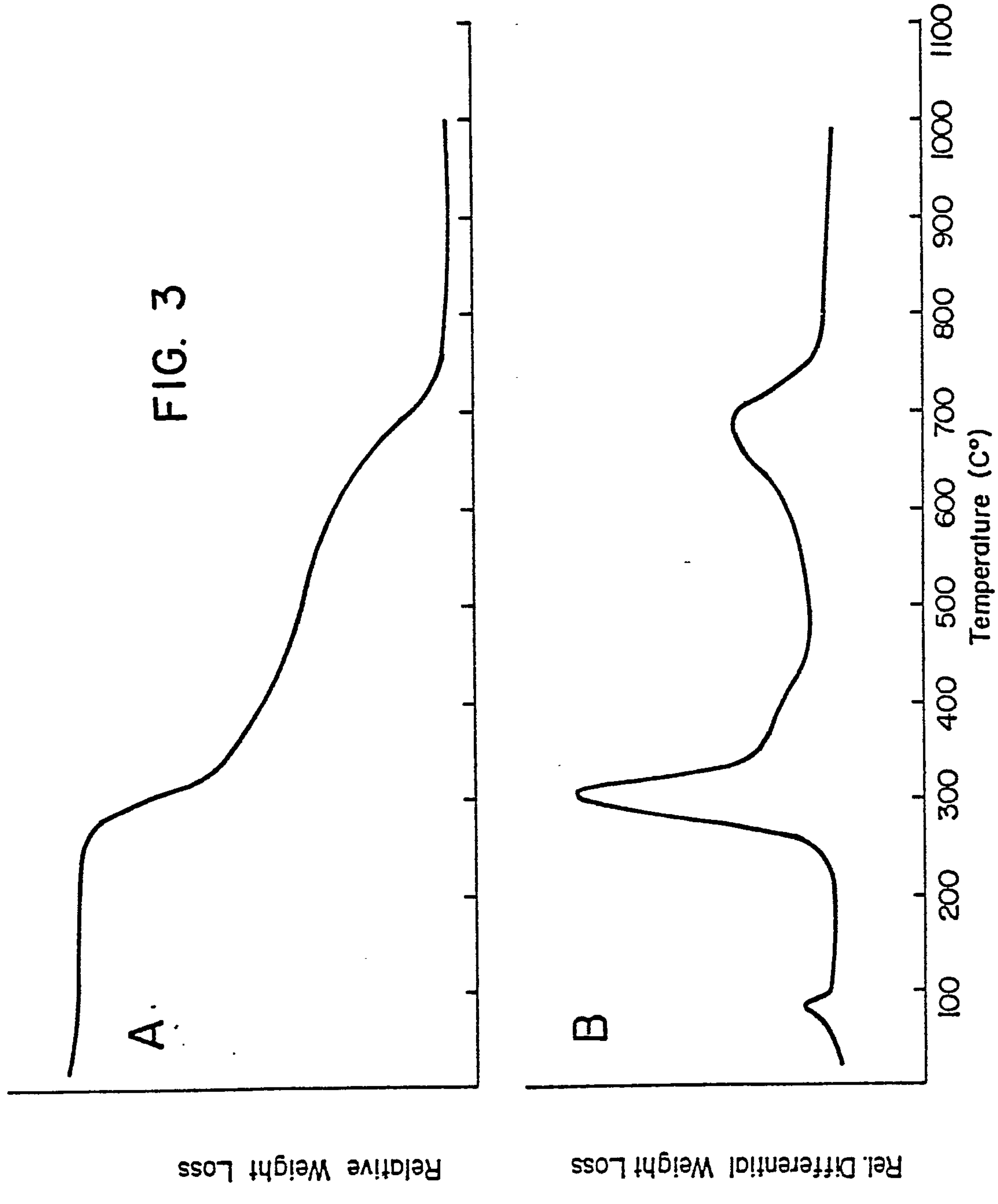
A
B
Relative Weight Loss
Rel. Differential Weight Loss
100
200
300
400
500
600
700
800
900
1000
1100
Temperature (C°)

FIG. 3